# EUROPÄISCHE PATENTANMELDUNG

(11) **EP 3 991 687 A1**
(43) Veröffentlichungstag der Anmeldung: **04.05.2022**
(21) Anmeldenummer: 21206069.3
(22) Anmeldetag: 02.11.2021
(51) Int. Cl.: A61C 5/00, A61C 13/10, A61C 13/15, A61N 5/06

(54) **SYSTEM AUS EINEM LASER UND EINEM ADHÄSIV ZUM BEFESTIGEN EINES DENTALRESTAURATIONSTEILS**

(30) Priorität: 02.11.2020 EP 20205294
(71) Anmelder: Ivoclar Vivadent AG, 9494 Schaan (LI)
(72) Erfinder: NIEDRIG, Christian, 9478 Azmoos (CH); BARBISCH, Michael, 6833 Klaus (AT); KÖHLER, Thomas, 78479 Reichenau (DE)
(74) Vertreter: Baronetzky, Klaus

(57) **Zusammenfassung**

Die Erfindung betrifft ein System aus einem Laser, der Laserstrahlung mit einer Wellenlänge von 1300 nm oder mehr, insbesondere zwischen 2750 nm und 3200 nm abgibt und mit einem Dental-Adhäsiv zur Aufbringung eines Dentalrestaurationsteils auf eine vorbehandelte, insbesondere gereinigte, Klebebasis, die durch Zahnhartgewebe oder durch Prothesenmaterial gebildet ist. Das auf die Klebebasis, welche frei von einem Dentalrestaurationsteil ist, aufgebrachte, Dental-Adhäsiv ist, insbesondere ohne Verblasen oder Agitieren, durch Applizieren von Laserstrahlung aktivierbar.

## Beschreibung

Die Erfindung betrifft ein System aus einem Laser und einem Adhäsiv, mit dessen Hilfe ein Dentalrestaurationsteil auf Zahnhartgewebe geklebt werden kann, gemäß dem Oberbegriff von Anspruch 1.

Die Aushärtung von Dentalmaterialien mit einem handhabbarem Lichthärtgerät mit mehreren Härtungs-Modi ist beispielsweise aus der WO 2016/202992 A1 bekannt.

Aus der US 2019/142700 A1 ist die Anwendung von Photonenenergie bekannt, um Dentalmaterialien mit Energie zu versorgen, um neben anderen Eigenschaften ihre physikalischen Handhabungseigenschaften, Wirksamkeit, Abgabefähigkeit, Reaktivität, Polymerisation und/oder mechanische Nachhärtungseigenschaften zu verbessern.

Für das Kleben von Dentalrestaurationsteilen wie Zähnen, Veneers, Inlays, Onlays oder einer Prothesenbasis auf Zahnhartgewebe (also Schmelz und/oder Dentin) wird ein spezielles Adhäsiv verwendet. Ein Beispiel hierfür ist das Adhäsiv "Adhese Universal" der Firma Ivoclar Vivadent AG, zu dem eine Verarbeitungsanleitung im Internet verfügbar ist.

Hier seien unter "Restaurationsteilen" aber auch erst durch Aushärtung zu diesen werdende Restaurationselemente wie Zahnfüllmaterialien, Komposits, Alkasite, Compomere, Glasionomerzemente usw. zu verstehen.

Dentaladhäsive sind selbsthärtend und/oder lichthärtend, weit überwiegend heutzutage lichthärtend, mit einem Empfindlichkeitsmaximum im Bereich sichtbaren Lichts, nämlich um 470 nm, bedingt durch den verwendeten Fotoinitiator Campherchinon, aber typischerweise auch mit UV-Licht-Anteilen.

Für die erfolgreiche Klebeverbindung ist ein sorgfältiger Auftrag des Adhäsiv auf das Zahnhartgewebe erforderlich. Das Adhäsiv muss für eine gute Verbindung in die Poren der Zahnsubstanz eindringen.

Zudem ist eine gleichmäßige Schichtdicke erforderlich. Typischerweise beträgt Schichtdicke des Adhäsivs weniger als 0,08 mm, bevorzugt weniger als 0,03 mm, besonders bevorzugt zwischen 0,01 bis 0,02 mm.

Gemäß Verarbeitungsanleitung des "Adhese Universal" ist es hierfür nötig, das Adhäsiv mittels einer Kleinbürste, einer sogenannten "micro brush", in die Zahnsubstanz einzumassieren oder einzurühren. Ein solcher Verarbeitungsschritt wird auch als Agitation bezeichnet.

Die Agitation muss über 15 bis 30 Sekunden, beispielsweise über 20 Sekunden, durchgeführt werden.

Für die Vergleichmäßigung der Schichtstärke wird gemäß der Verarbeitungsanleitung anschließend ein Verblasen mittels Druckluft vorgenommen.

Auch hierfür sind einige Sekunden zu rechnen, und zudem muss an der Stelle, an der Bearbeitung stattfindet, Druckluft zur Verfügung stehen.

Das Verfahren ist vergleichsweise umständlich und zeitaufwändig. Dennoch wird dieses mehrschrittige und zeitaufwändige Verfahren seit Jahrzehnten bei unterschiedlichen Adhäsiven angewendet.

Daher liegt der Erfindung die Aufgabe zugrunde, ein System aus einem Laser und einem Adhäsiv gemäß dem Oberbegriff von Anspruch 1 zu schaffen, das zum einen hinsichtlich des Prozessablaufs verbessert und universeller anzuwenden ist, und zum anderen hinsichtlich der Akzeptanz sowohl bei Zahnärzten als auch bei deren Patienten verbessert ist.

Diese Aufgabe wird erfindungsgemäß durch Anspruch 1 gelöst. Vorteilhafte Weiterbildung ergeben sich aus den Unteransprüchen.

Erfindungsgemäß ist es vorgesehen, vor dem Aufbringen des Dentalrestaurationsteils, in einem zusätzlichen Schritt, das zuvor auf einer Klebebasis aufgetragene Dental-Adhäsiv einer Laserbehandlung zu unterziehen. Als Adhäsiv kann erfindungsgemäß z.B. das bereits erwähnte "Adhese Universal" verwendet werden, aber auch ein beliebiges anderes geeignetes Dentaladhäsiv. Bevorzugt enthält das Adhäsiv meist Mischungen verschiedener Methacrylate in Lösemitteln (z.B. Ethanol, Aceton). Die Monomere können eine oder mehrere Methacrylatgruppen und weitere funktionelle Gruppen enthalten. Weitere funktionelle Gruppen dienen der Bereitstellung besonderer Produkteigenschaften, wie z.B. Carbonsäure- oder Phosphorsäureestergruppen, der Selbstätzung und der Haftvermittlung auf Zahnhartgewebe. Die Adhäsive können zusätzlich Verarbeitungshilfsmittel (z.B. Rheologieadditive zum Erreichen der typischen Viskosität von Dentaladhäsiven), Stabilisatoren (Radikalfänger), sowie Photoinitiatoren und ggf. Teile von Redoxinitiatorsystemen enthalten für die Licht- und/oder Dualhärtung und/oder Selbsthärtung.

Die Klebebasis kann ein Zahnhartgewebe wie Dentin oder Schmelz sein, aber beispielsweise auch ein Dentalrestaurationsteil wie ein künstlicher Zahn, beispielsweise aus Keramik, oder eine Prothesenbasis, beispielsweise aus PMMA. Auch sind andere Dentalrestaurationsteile wie beispielsweise eine Krone, eine Teilkrone, ein Inlay oder ein Onlay z.B. aus Glaskeramik, Oxidkeramik oder CAD-Composit als Klebebasis möglich.

Das erfindungsgemäße System aus einem Laser und einem Adhäsiv kann innerhalb oder außerhalb des Mundes eines Patienten verwendet werden. Bevorzugt wird es innerhalb des Mundes des Patienten verwendet.

Überraschend lässt sich mit der Maßnahme, mittels eines Bestrahlungslasers mit einer vergleichsweise hohen Wellenlänge im Infrarotbereich, wie um 1580 nm oder um 3000 nm, eine Bestrahlung anzuwenden, eine erhebliche Zeitersparnis erzielen. Dies ist überraschend, denn es würde erwartet, dass ein zusätzlicher Schritt den Verfahrensablauf verlängert.

Durch die Beaufschlagung des auf die Klebebasis aufgebrachten Adhäsivs mit Laserstrahlung wird dieses vorkonditioniert bzw. aktiviert. Diese Aktivierung führt zu einer Verbesserung der Haftfähigkeit, und damit zu einer Verbesserung der Scherfestigkeit der Befestigung des auf den Zahn später aufgebrachten Restaurationsteils.

Das Adhäsiv wird durch die erfindungsgemäße Beaufschlagung mit Laserstrahlung, also die Aktivierung, in einen Zustand versetzt, in dem es für die umgehende Aufbringung des Dentalrestaurationsteils bereit ist. Dies erlaubt erfindungsgemäß sowohl, den Schritt des mindestens 20 Sekunden dauernden Agitierens des aufgebrachten Adhäsivs, als auch, den Schritt des Verblasens, überflüssig zu machen. Gerade bei Anwendung im Mund des Patienten entfällt damit auch die Belästigung des Patienten durch die lange Agitationszeit.

Die Akzeptanz ist insofern erhöht, insbesondere, wenn auch auf den Schritt des Verblasens verzichtet wird. Gerade empfindliche Patienten, , die beispielsweise unter Dentin-Hypersensibilität leiden, empfinden die intensive Zuführung von Kaltluft häufig als unangenehm.

Zudem wird die turbulente Anströmung bzw. Überströmung von wasserhaltigem Weichgewebe oder Flüssigkeiten durch Aerosole oder Tröpfchen gerade in Zeiten von Epidemien als riskant angesehen, da sie die Möglichkeiten von Infektionen erhöhen.

Erfindungsgemäß kann der die Laserstrahlung abgebende Laser, der Behandlungslaser, in an sich bekannter Weise entweder als Laser mit Netzanschluss oder gegebenenfalls mit Akkumulatorbetrieb ausgebildet sein. Als Behandlungslaser kann auch ein Bakterienentfernungslaser verwendet werden, insbesondere ein solcher gemäß der EP 3 628 266 A1. Auf diese Offenlegungsschrift wird vollinhaltlich Bezug genommen.

In einer weiteren Ausführungsform wird zusätzlich zu dem Behandlungslaser eine Lichtquelle verwendet. Diese beiden Strahlungsquellen haben bevorzugt unterschiedliche Leistungspegel. Der Behandlungslaser mit dem ersten Leistungspegel dient der Aktivierung des Adhäsivs, das auf die Klebebasis aufgebracht ist, bevor das Dentalrestaurationsteil aufgebracht wird.

Anschließend an diesen Aktivierungsschritt des auf die Klebebasis aufgebrachten Adhäsivs wird bei direkten Restaurationen das Komposit als Dentalrestaurationsteil, insbesondere stopfbar oder fließfähig, aufgebracht. Bei dem indirekten Aufbringen von Restaurationen wird zwischen dem Adhäsiv, welches auf die Klebebasis und/oder auf die Restauration, insbesondere einer Krone, aufgebracht ist, und der Restauration ein Zement aufgebracht.

In einem weiteren Schritt wird, insbesondere nach Aufbringen eines Zementes, das Dentalrestaurationsteil aufgebracht und das Adhäsiv, welches sich nun zwischen Klebebasis und Dentalrestaurationsteil befindet, durch die Lichtquelle, also die zweite Strahlungsquelle mit dem zweiten Leistungspegel ausgehärtet. Hierbei ist es vorteilhaft, wenn die zweite Strahlungsquelle eine für die Polymerisation des Adhäsivs geeignete Wellenlänge emittier. Der Leistungspegel eines üblichen Dentallichthärtgeräts als zweite Strahlungsquelle beträgt -über die Zeit gemittelt z.B. 1200-3000 mW/cm2.

Zwar sind Er:YAG-Festkörperlaser in der Regel als Standgerät ausgebildet und verfügen über einen Netzanschluss. Typischerweise sind jedoch an dem Standgerät Rollen angebracht, so dass der Laser verfahrbar ist und insofern die Mobilität im Vergleich mit einer (in der Regel stationären) Druckluftquelle, wie sie für das Verblasen erforderlich ist, verbessert ist. Zudem sind mittlerweile Er:YAG-Laser der Größe von wenigen Kubikdezimetern entwickelt worden, was der Mobilität weiter zugute kommt; die Entwicklung hinsichtlich der Verkleinerung schreitet zudem weiter voran.

Das erfindungsgemäße System aus einem Laser und einem Adhäsiv ist zudem unabhängiger als die zuvor bekannten Systeme, da es nicht von einer Druckluftquelle abhängig ist, wenn bei dessen Anwendung auf den Schritt des Verblasens verzichtet wird.

In modifizierter Ausgestaltung der Erfindung ist es jedoch nicht ausgeschlossen, dass ein kurzes Zusatz-Verblasen erfolgt, entweder vor oder nach dem dritten Schritt, also dem Laserbehandlungsschritt.

Während in der Fachliteratur seit Jahrzehnten darauf hingewiesen wird, wie wichtig das Agitieren und Verblasen im Hinblick auf die erforderliche Eindringtiefe des Dentaladhäsivs und die Vergleichmäßigung der Schichtdicke ist, zeigt sich erfindungsgemäß überraschend, dass die Scherfestigkeit einer mit dem erfindungsgemäßen System aufgebrachten Dentalrestauration sogar noch höher sein kann, als bei einer Referenzrestauration, die in klassischer Weise, also mittels Agitierens und Verblasens, aufgebracht wurde.

Bevorzugt belaufen sich die Werte der Scherfestigkeit, also die Scherhaftwerte, auf Werte oberhalb folgender Grenzen:

Die Scherfestigkeit des Adhäsivs, wie beispielsweise "Adhese Universal" im Self-Etch Modus, auf bovinem Substrat sollte bei Dentin mindestens 25 MPa und bei Schmelz mindestens 17 MPa betragen. Ein Adhäsiv im "Self-Etch Modus" bedeutet in diesem Fall, dass es einen Primer, ein Methacrylat Monomer und andere Polymerkomponenten mit einem sauren pH-Wert enthält, der in der Lage ist, Schmelz und Dentin zu demineralisieren und gleichzeitig in diese konditionierten Oberflächen einzudringen.

Bei einer auf Zahnschmelz aufgebrachten erfindungsgemäßen Dentalrestauration ließ sich bei einer Bestrahlungszeit des Adhäsiv-Behandlungslasers von 1 Sekunde eine Scherfestigkeit von mehr als 22 MPa erzielen.

Bei einer auf Dentin aufgebrachten Dentalrestauration ließ sich bei einer erfindungsgemäßen Bestrahlungszeit des Behandlungslasers von 5 Sekunden eine Scherfestigkeit von mehr als 34 MPa erzielen.

Die mit dem erfindungsgemäßen System aufgebrachten Dentalrestaurationen zeigen somit hohe Scherfestigkeiten, und dies trotz eines verkürzten Verfahrensablaufs im Vergleich zum Stand der Technik.

In beiden Fällen war die Festigkeit gleich gut oder besser als bei einer als Referenzmuster mittels Agitierens und Verblasens des Adhäsivs aufgebrachten Dentalrestauration (22 MPa bzw. 33 MPa).

Gerade bei Anwendung auf Zahnschmelz als Klebebasis ergibt sich so eine signifikante Zeitersparnis von etwa 20 Sekunden pro Dentalrestauration.

Bei dem erfindungsgemäßen System aus einem Laser und einem Adhäsiv zum Befestigen eines Restaurationsteils, nämlich eines Dentalrestaurationsteils, ist es vorgesehen, dass zunächst die Klebebasis, also beispielsweise die Zahnsubstanz, in einem Vorbehandlungsschritt vorbehandelt wird. Dies kann dadurch erfolgen, dass ein mechanisches Abtragen von kariöser Zahnhartsubstanz, gefolgt von einem Wasserspray-Einsatz zum Reinigen von Dentin-/Smearlayer/ Schmelzmaterial, vorgenommen wird.

Hierzu kann aber auch ein Ätzschritt, beispielsweise durch Ätzen mit Phosphorsäure, gehören, der allerdings bei Verwendung von Universaladhäsiven entfallen kann.

In vorteilhafter Ausgestaltung der Erfindung kann auch vor dem Aufbringen des Adhäsivs zusätzlich eine Laserbeaufschlagung mit dem Behandlungslaser mit einem Emissionswellenlängenbereich von mehr als 1300 nm eingesetzt werden.

In an sich bekannter Weise wird in einem Auftragsschritt das Adhäsiv aufgetragen, bevorzugt mittels eines professionellen Applikators wie einer Mikrobürste.

In dem dritten Schritt, dem Laserbehandlungsschritt, wird erfindungsgemäß das aufgetragene Adhäsiv einer Laserbehandlung mit Laserstrahlung mit einem Emissionsmaximum von beispielsweise zwischen 1400 nm und 1600 nm oder von etwa 2940 nm unterzogen. Bei diesen Wellenlängen bestehen Absorptionsbande von Wasser.

Überraschend ergibt sich durch diese Laserbehandlung der - gleichsam rohen - Adhäsivschicht eine Konditionierung oder Aktivierung des Adhäsivs, die dazu führt, dass die Scherfestigkeit im Vergleich zu dem Verfahren ohne diesen genannten Schritt deutlich erhöht ist. Es ergibt sich also, dass ohne ein Verblasen oder Agitieren nur durch die Verwendung der erfindungsgemäßen Kombination aus einem Behandlungslaser mit einem Adhäsiv eine überraschend hohe Scherfestigkeit erreichbar ist. Das zeitaufwändige Verblasen und Agitieren kann enrfindungsgemäß entfallen.

Eine mögliche Erklärung für diese überraschende Wirkung ist, dass durch den Behandlungslaser der Wasseranteil und/oder leicht flüchtige organische Komponenten aus den Tubuli des Dentins entfernt werden, so dass das Adhäsiv in die Tubuli eindringen, bzw. besser hineindiffundieren kann, und dadurch die Retention besser wird.

Soweit Zahnschmelz betroffen ist, ist eine weitere mögliche Erklärung, dass die Temperaturerhöhung, die sich bei Anwendung des Lasers durch das gesamte Adhäsiv hindurch erfindungsgemäß einstellt, zu einer stärkeren Ätzwirkung des sauren Monomers führt.

In vorteilhafter Ausgestaltung kann damit bei einer Anwendung des erfindungsgemäßen Systems sowohl der Agitationsschritt als auch der Verblasschritt entfallen. Aber auch wenn diese beiden Zusatzschritte vorgenommen werden, wie es gemäß dem klassischen Befestigungsverfahren von Dentalrestaurationsteilen der Fall ist, ergibt sich mit dem erfindungsgemäßen System aus einem Laser und einem Adhäsiv eine im Wesentlichen gleich gute oder sogar bessere Scherfestigkeit.

In einem Aufbringschritt wird das Dentalrestaurationsteil auf das - konditionierte bzw. aktivierte - Adhäsiv aufgebracht und in an sich bekannter Weise aufgedrückt.

In ebenfalls an sich bekannter Weise wird daraufhin für eine Polymerisation oder Aushärtung des Adhäsivs mittels der zweiten Strahlungsquelle gesorgt; bei Lichtpolymerisation typischerweise mit einer Wellenlänge zwischen 400 und 480 nm. So wird eine sichere Befestigung des Dentalrestaurationsteils sichergestellt.

In an sich bekannter Weise sind Dentaladhäsive auch geeignet, hydrophile Klebeverbindungen bereitzustellen, also sowohl an Dentin als auch an Zahnschmelz anzukoppeln. Dentin hat bekanntlich ein etwas höheren Wasseranteil als Zahnschmelz, von zum Beispiel etwa 10 %. Möglicherweise ist dies die Erklärung für die Ergebnisse von Versuchsreihen, die in Zusammenhang mit dem erfindungsgemäßen System aus einem Laser und einem Adhäsiv vorgenommen wurden, aus denen sich das Ergebnis ergibt, dass in der Tendenz bei Dentin eine längere Bestrahlungszeit mit dem Behandlungslaser als bei Zahnschmelz zu der erwünschten hohen Scherfestigkeit führt.

Es versteht sich, dass die optimale Bestrahlungszeit in weiten Bereichen an die Erfordernisse angepasst werden sollte.

Auch bei einer längeren Bestrahlungszeit des Adhäsivs von 5 Sekunden mit dem erfindungsgemäßen Behandlungslaser ergibt sich eine signifikante Zeitersparnis gegenüber der klassischen Agitationszeit von 20 bis 30 Sekunden.

Bei Klebefugen, die sowohl Dentin als auch Zahnschmelz betreffen, ist es bevorzugt, eine längere Bestrahlungszeit des Adhäsivs von beispielsweise 5 Sekunden zu verwenden, die für Dentin optimal ist und beim Zahnschmelz eine nur um weniges geringere Scherfestigkeit ermöglicht.

Erfindungsgemäß gibt der Laser des erfindungsgemäßen Systems aus einem Laser und einem Adhäsiv Strahlung in einem Wellenbereich zwischen 1300 nm und 4500 nm ab. Die Strahlung aus dem Laser wird dem freiliegenden Adhäsiv zugeleitet, das auf die Klebebasis aufgebracht ist. Die Strahlung durchdringt das Adhäsiv und dringt in die Klebebasis ein.

Bevorzugt ist eine Steuervorrichtung für diesen Behandlungslaser vorgesehen, mittels der eine Bestrahlungszeit einstellbar ist oder fest eingestellt ist. Durch die Strahlung des Behandlungslasers wird das auf der Klebebasis aufgebrachte Adhäsiv so konditioniert, dass es bei Verwendung für die Befestigung von Dentalrestaurationen zu einer hohen Scherfestigkeit der Klebeverbindung führt.

Es versteht sich, dass hierfür zwischen der Strahlungsaustrittsfläche des Behandlungslasers und der Oberfläche des Adhäsivs keine die Bestrahlung reduzierenden Objekte, wie beispielsweise ein aufgebrachtes Dentalrestaurationsteil, vorhanden sein sollten.

Bei dem erfindungsgemäßen System ist es also vorgesehen, dass der Behandlungslaser das freiliegende Adhäsiv direkt beaufschlagt.

Der erfindungsgemäße Behandlungslaser hat bevorzugt einen Lichtleiterstab mit abgekröpftem distalen Ende. An dessen Strahlungsaustrittsfläche wird die Laserstrahlung abgegeben.

Bevorzugt ist der Lichtleitstab an einem Handstück angebracht, das entweder die Strahlungsquelle des Behandlungslasers aufweist, oder über einen biegsamen Lichtleiter mit einem Standgerät verbunden ist, das die Strahlungsquelle enthält.

Für die Aufbringung der erfindungsgemäßen Adhäsiv-Behandlungsstrahlung wird das Handstück bevorzugt vom Zahnarzt in eine Stellung gebracht, in welcher sich die Strahlungsaustrittsfläche oberhalb des Adhäsivs befindet, bevorzugt in einer solchen Position, dass die Strahlungsaustrittsfläche sich im wesentlichen parallel zu der Adhäsivschicht erstreckt.

Auch wenn erfindungsgemäß bevorzugt ein Festkörperlaser verwendet wird, der zudem bevorzugt im Pulsbetrieb arbeitet, ist es auch möglich, anstelle dessen einen preisgünstigeren Diodenlaser, welcher bevorzugt ohne Pulsbetrieb, also kontinuierlich, arbeitet, zu verwenden. Der Diodenlaser kann auch kontinuierlich Behandlungslaserstrahlung emittieren. Bei einem derartigen Diodenlaser ist es möglich, das bei einem Wert zwischen 1400 nm und 1600 nm liegende Absorptionsmaximum von Wasser auszunutzen. Ein Diodenlaser hat gegenüber einem Er:YAG-Laser den Vorteil, dass er er deutlich kompakter als dieser und daher besser handhabbar ist.

Auch besitzt er eine höhere Lebensdauer, oft mehr als 30.000 Stunden, als andere Lasertypen, wie beispielsweise ein Er:YAG-Laser.

Auch sind Diodenlaser sehr energieeffizient und besitzen einen vergleichsweise hohen elektrisch/optischen Wirkungsgrad von 25 bis über 50 %. Ferner ist auch ihre geringe Leistungsdegradation von weniger als 1%/1000h bei Betrieb mit Nennstrom ein enormer Vorteil gegenüber anderen Lasertypen.

In einer Ausführungsform des erfindungsgemäßen Systems wird als Laser ein Diodenlaser verwendet, der in Bereich von 1400 nm bis 1600 nm emittiert, also in einem Bereich, in dem Absorptionsbande von Wasser vorliegen.

Weitere Vorteile, Einzelheiten und Merkmale ergeben sich aus der nachfolgenden Beschreibung mehrerer Ausführungsformen der Erfindung anhand der Zeichnungen.

Es zeigen:
- Fig. 1: eine schematische perspektivische Ansicht eines Systems aus Laser und Adhäsiv, in einer Ausführungsform der Erfindung, zur Erläuterung des erfindungsgemäßen System aus einem Laser und einem Adhäsiv;
- Fig. 2: ein Detail einer weiteren Ausführungsform eines erfindungsgemäßen Systems aus einem Festkörperlaser und einem Adhäsiv;
- Fig. 3: eine schematische Darstellung einer Aushärtung des Adhäsivs nach Aufbringen des Dentalrestaurationsteils auf das Adhäsiv;
- Fig. 4: eine Grafik zur Darstellung der Scherfestigkeit, die bei unterschiedlichen Bestrahlungszeiten bei der Ausbildung der Klebebasis als Schmelz sowie unter Verwendung eines gepulsten Lasers erreichbar ist;
- Fig. 5: eine Grafik zur Darstellung der Scherfestigkeit, die bei unterschiedlichen Bestrahlungszeiten bei der Ausbildung der Klebebasis als Dentin sowie unter Verwendung eines gepulsten Lasers erreichbar ist;
- Fig. 6: eine Grafik zur Darstellung der Scherfestigkeit, die bei unterschiedlichen Bestrahlungszeiten bei der Ausbildung der Klebebasis als Schmelz sowie unter Verwendung eines nichtgepulsten Lasers erreichbar ist;
- Fig. 7: eine Grafik zur Darstellung der Scherfestigkeit, die bei unterschiedlichen Bestrahlungszeiten bei der Ausbildung der Klebebasis als Dentin sowie unter Verwendung eines nichtgepulsten Lasers erreichbar ist; und
- Fig. 8 und 8a: eine weitere Ausführungsform eines Behandlungs-Lasers mit einer Spitze, die besonders für die Ausführung des Laserbehandlunsgschritts in tiefen Kavitäten geeignet ist.

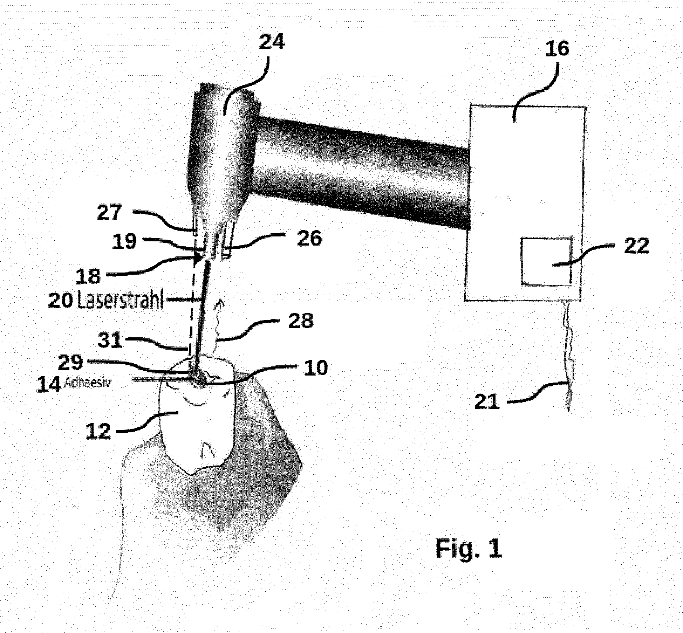

Eine Ausführungsform eines erfindungsgemäßen Systems wird anhand von Fig. 1 beschrieben.

Fig. 1 zeigt eine Klebebasis 10 auf bzw. an einem Zahn 12, im dargestellten Ausführungsbeispiel einem Molar oder Prämolar mit einer Kavität in der Okklusalfläche.

Zur Vorbehandlung wird in an sich bekannter Weise ein Fräsen, Schleifen und/oder Polieren des Zahnhartgewebes vorgenommen, bis ausschließlich gesundes Gewebe vorliegt.

Bei Bedarf kann in an sich bekannter Weise zur Vorbehandlung ein Ätzmittel aufgebracht werden.

In ebenfalls an sich bekannter Weise wird ein Adhäsiv 14 auf die Klebebasis 10 aufgetragen.

Mittels einer Aufstreichvorrichtung, wie beispielsweise mittels einer Microbrush oder eines anderen geeigneten Dental-Applikators wird das aufgebrachte Adhäsiv 14 zu einer Schicht von unter 0.03 mm verteilt.

Dieses Auftragen stellt den Auftragsschritt, den zweiten Schritt, dar.

In einem dritten Schritt, dem Laserbehandlungsschritt, wird ein Behandlungs-Laser 16 mit seiner Strahlungsaustrittsfläche 18 an einem Lichtleitstab als mögliches Ausführungsbeispiel eines Lichtleiters 19 so ausgerichtet, dass der austretende Laserstrahl, der die Strahlung 20 bildet, auf die Adhäsivschicht 14 gerichtet ist. Der Laser 16 wird eingeschaltet, und dadurch erfolgt eine Laserbehandlung der Adhäsivschicht.

Der Laser 16 ist in dem dargestellten Ausführungsbeispiel ein Festkörperlaser, nämlich ein Er:YAG-Laser. Er hat ein Emissionsmaximum bei einer Wellenlänge von 2940 nm und einen Netzanschluss 21. Bevorzugt hat der Er:YAG-Laser eine elektrische Leistung zwischen 0,4 Watt und 10 Watt, bevorzugt zwischen 1 Watt und 2 Watt und besonders bevorzugt von 1,3 Watt.

Die Klebebasis 10 besteht in dem dargestellten Ausführungsbeispiel zum größeren Teil aus Zahnschmelz und zum kleineren Teil aus Dentin. Es ist wichtig, dass das Adhäsiv 14 überall eine gute Scherfestigkeit zeigt.

Es wird beispielsweise eine Bestrahlungszeit von 5 Sekunden von einer Steuervorrichtung 22 vorgegeben, und dementsprechend wird der Laser 16, welcher als gepulster (Versuche 1 und 2) oder kontinuierlicher (Versuche 3 und 4) Laser ausgestaltet sein kann, für 5 Sekunden eingeschaltet.

Durch diese Laserbelichtung mit der Laser-Strahlung 20 in dem genannten Wellenlängenbereich ist das Adhäsiv 14 so konditioniert, dass es nach der Applikation des Dentalrestaurationsteils und nach der Polymerisation des Adhäsivs, also nach Fertigstellung, eine hohe Scherfestigkeit hat. Nur durch dieses erfindungsgemäße System aus Behandlungslaser und Adhäsiv lässt sich zeitsparend die erwünschte überraschend hohe Scherfestigkeit erreicht werden.

Bei den ersten beiden Ausführungsbeispielen (Versuch 1 und 2) wird ein gepulster Laser mit einem Pumpstrom von 300 Ampere mit einer Pulsdauer von 300 Mikrosekunden, einer Pulsfrequenz von 150 Hz sowie einer mittleren, also über die Impulsfolge gemittelten Laserleistung von 1,3 W verwendet. Der Strahldurchmesser beträgt 5,4 mm.

Es wurden insgesamt vier Versuche mit Ausführungsformen unter Verwendung des erfindungsgemäßen Systems aus einem Laser und einem Adhäsiv vorgenommen.

Beim ersten Versuch wurde als Klebebasis 10 boviner Zahnschmelz gewählt. Der Versuch wurde mit unterschiedlichen Bestrahlungszeiten durch den gepulsten Laser wiederholt, und es ergaben sich die folgenden Scherfestigkeitswerte, gemäß der nachstehenden Tabelle 1 (nach ISO 29022):

**Tabelle 1: (Versuch 1)**

| Bestrahlung / s | 10 | 7.5 | 5 | 2.5 | 1 | 0 | n/a (Referenz nach Standard) |
|---|---|---|---|---|---|---|---|
| *Anzahl Versuche* | 5 | 5 | 20 | 5 | 5 | 4 | 5 |
| Mittelwert Scherfestigkeit in MPa | 18.38 | 21.75 | 20.22 | 21.47 | 22.94 | 19.11 | 22.09 |
| *Standardabweichung* | 2.64 | 7.69 | 4.84 | 2.46 | 6.57 | 1.31 | 2.67 |
| Min | 14.93 | 15.16 | 11.57 | 17.8 | 19.25 | 17.83 | 19.64 |
| Max | 21.3 | 32.81 | 31.45 | 23.62 | 34.61 | 20.71 | 25.56 |
| Bruchbild | adhäsiv | adhäsiv | adhäsiv | adhäsiv | adhäsiv | adhäsiv | adhäsiv |

Das Bruchbild kann - wie in der Tabelle - adhäsiv oder aber auch kohäsiv sein. Ein Adhäsionsbruch ist ein Bruch, der exakt entlang der Phasengrenzfläche zwischen Fügeteiloberfläche und Klebstoff verläuft. Es kommt bei dieser Bruchart zu einer vollständigen Trennung des Klebstoffs von der bzw. den Substratoberflächen.

Ein Kohäsionsbruch bedeutet, dass die Adhäsion des Klebstoffs an den Phasengrenzen größer als die Bruchfestigkeit der Klebebasis ist. Die Klebefuge übersteht also den Bruchtest, und das Substrat bricht anstelle dessen.

Der Versuch wurde mit bovinem Dentin als Klebebasis 10 wiederholt.

Es ergaben sich die aus der nachstehenden Tabelle 2 ersichtlichen Scherfestigkeitswerte (nach ISO 29022):

**Tabelle 2: (Versuch 2)**

| Belichtung / s | 10 | 7.5 | 5 | 2.5 | 1 | 0 | n/a (Referenz nach Standard) |
|---|---|---|---|---|---|---|---|
| *Anzahl Versuche* | 5 | 5 | 20 | 5 | 5 | 5 | 5 |
| Mittelwert Scherfestigkeit in MPa | 29.27 | 31.21 | 34.19 | 30.80 | 17.89 | 18.47 | 33.08 |
| *Standardabweichung* | 5.18 | 3.65 | 7.59 | 2.07 | 3.36 | 1.92 | 3.89 |
| Min | 22.93 | 26.98 | 20.46 | 27.59 | 13.31 | 16.18 | 26.69 |
| Max | 36.72 | 36.15 | 46.56 | 32.97 | 22.10 | 21.38 | 37.31 |
| Bruchbild | 3/5 kohäsiv | 4/5 kohäsiv | 19/20 kohäsiv | kohäsiv | adhäsiv | adhäsiv | kohäsiv |

Die Versuche 1 und 2 wurden unter Verwendung von gepulster Laser-Strahlung und des erfindungsgemäßen Systems aus einem Laser und einem Adhäsiv durchgeführt.

Ferner wurden Versuche 3 und 4 unter Verwendung eines kontinuierlich betriebenen, also ungepulsten Diodenlasers unternommen. Deren Ergebnisse sind aus den nachstehenden Tabellen ersichtlich.

Beim Versuch 3 wurde als Klebebasis 10 wiederum boviner Zahnschmelz gewählt. Der Versuch wurde mit unterschiedlichen Bestrahlungszeiten des kontinuierlich betriebenen Diodenlasers durchgeführt. Die Daten, welche mit der Er:YAG-Laserbestrahlung im Pulsmodus erzielt wurden, sind zu Referenzzwecken ebenso in Tabelle 3 angegeben.

Ferner sind auch die Werte angegeben, welche sich ohne Laserbestrahlung ergeben. Und zusätzlich sind auch Referenzwerte angeführt, die sich ergeben, wenn nach der Standardprozedur mit Verblasen und Agitieren gearbeitet wird. Es ergaben sich die folgenden Scherfestigkeitswerte, gemäß der nachstehenden Tabelle 3 (nach ISO 29022):

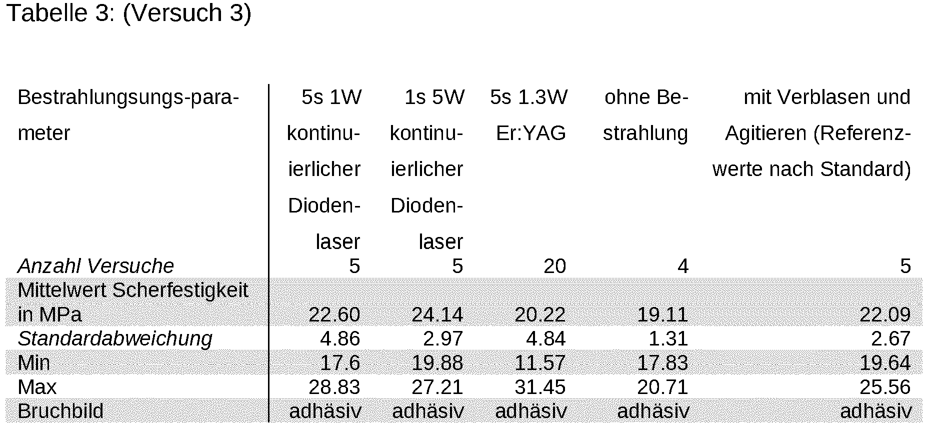

Versuch 4 wurde mit bovinem Dentin als Klebebasis 10 vorgenommen, also wie Versuch 2, jedoch unter Verwendung eines kontinuierlich betriebenen Diodenlasers.

Es ergaben sich die aus der nachstehenden Tabelle 4 ersichtlichen Scherfestigkeitswerte (nach ISO 29022):

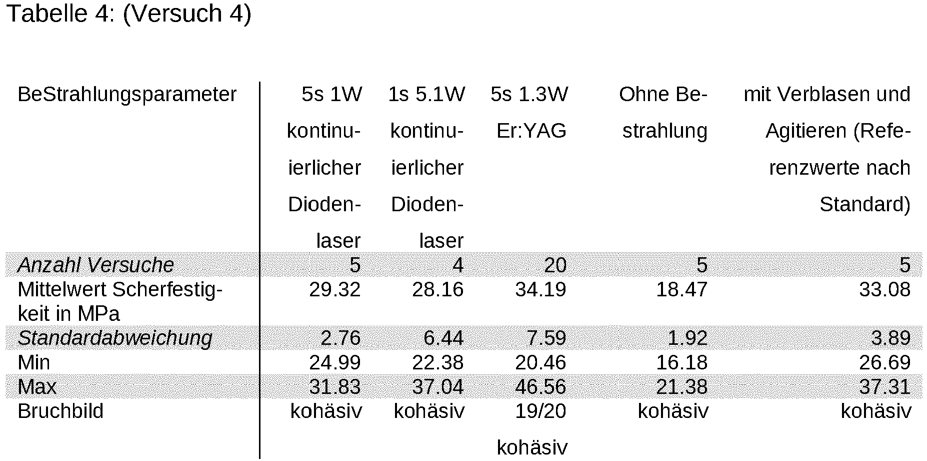

Durch diese beiden Versuche unter Verwendung eines kontinuierlich betriebenen Diodenlasers (Versuch 3 und 4) konnte gezeigt werden, dass die zeitsparende Verwendung des erfindungsgemäßen Systems aus Laser und Adhäsiv nicht auf die Verwendung von gepulster Laserstrahlung beschränkt ist und nicht auf Kosten der Scherfestigkeit geht.

Weiterhin untermauern die Ergebnisse der erfindungsgemäßen Versuche sowohl mit dem kontinuierlich betriebenen Diodenlaser als auch mit dem gepulsten Laser, dass erfindungsgemäß eine ähnlich hohe oder sogar teils eine höhere Scherfestigkeit erreicht wird wie bei der Standardprozedur durch Agitation und Verblasen.

Die erfindungsgemäße Wirkung kann sich dadurch erklären lassen, dass der Behandlungslaser den Wasseranteil und/oder leicht flüchtige organische Komponenten aus den Tubuli des Dentins oder den Poren des Zahnschmelzes entfernt. Durch den entstandenen Platz in den Tubuli bzw. den Poren kann nun das Adhäsiv in die Tubuli eindringen, bzw. besser hineindiffundieren. Als Folge dieses Eindringens des Adhäsivs wird die Retention und somit auch die Scherfestigkeit erhöht.

Zahnhartgewebe hat andere Reflektionseigenschaften als ein aufgebrachtes Adhäsiv 14. Um die Ausrichtung des Handstücks 24 des Lasers 16 zu erleichtern, ist an dem Handstück 24 der Strahlungsaustrittsfläche 18 benachbart ein Sensor 26 vorgesehen, der die reflektierte Strahlung 28 erfasst und der Steuervorrichtung 22 zuleitet.

Bevorzugt lässt sich hiermit ein Signal angeben, dass die richtige Ausrichtung des Handstücks 24 signalisiert.

Das Handstück 24 weist auch eine Ziel-Lichtquelle auf, die bevorzugt als Ziellaser 27 ausgebildet ist, und sichtbares Licht, z. B. im blauen, grünen, gelben oder roten Wellenlängenbereich emittiert. Es ist auch weißes oder ein anderes Mischlicht möglich. Der Ziellaser 27 ist physikalisch mit dem Behandlungs-Laser 16 verbunden. Er ist so ausgerichtet, dass sein Zielstrahl 31 auf einen Fokusfleck 29 des Behandlungs-Lasers 16 trifft. Dort erzeugt er einen sichtbaren Lichtpunkt. Er ist demnach zu diesem achsparallel, oder im wesentlichen achsparallel ausgerichtet.

Die Achse des Ziellasers 27 kann aber auch - wie in Fig. 1 dargestellt - leicht zur Achse des Behandlungs-Lasers 16 geneigt sein.

Mit dem Ziellaser 27 kann die Ausrichtung des Behandlungs-Lasers 16 kontrolliert und ggf. nachjustiert werden. Er wird bevorzugt eingeschaltet, bevor der Laser 16 eingeschaltet wird. Das Handstück 24 wird auf die erwünschte Stelle ausgerichtet, und dann wird der Behandlungs-Laser 16 eingeschaltet.

Der Ziellaser kann auch im Lasergehäuse 16 integriert sein und das sichtbare Licht direkt in den Lichtwellenleiter 19 des Lasers einkoppeln 19, so dass keine "eigene" Strahlungsführung nötig ist. Dies hat den Vorteil, dass das Ziellicht die exakt selbe Optik verwendet und damit (abgesehen ggf. von chromatischer Aberration) die tatsächlich beleuchtete Fläche recht gut repräsentiert - unabhängig vom Abstand zur Behandlungsfläche und von eventuellen trigonometrischen Überlegungen bei unterschiedlichen Austrittsöffnungen.

Insofern sei auf die übliche Bauart von Lasersystemen mit integriertem Ziellaser verwiesen. Jedoch muss nicht ein Laser als Ziel-Lichtquelle eingesetzt werden, da Kohärenz hier nicht notwendig ist, nur eine sichtbare Beleuchtung. Daher ist prinzipiell jede sichtbares Licht abgebende Lichtquelle zur Erzeugung des Zielstrahls geeignet.

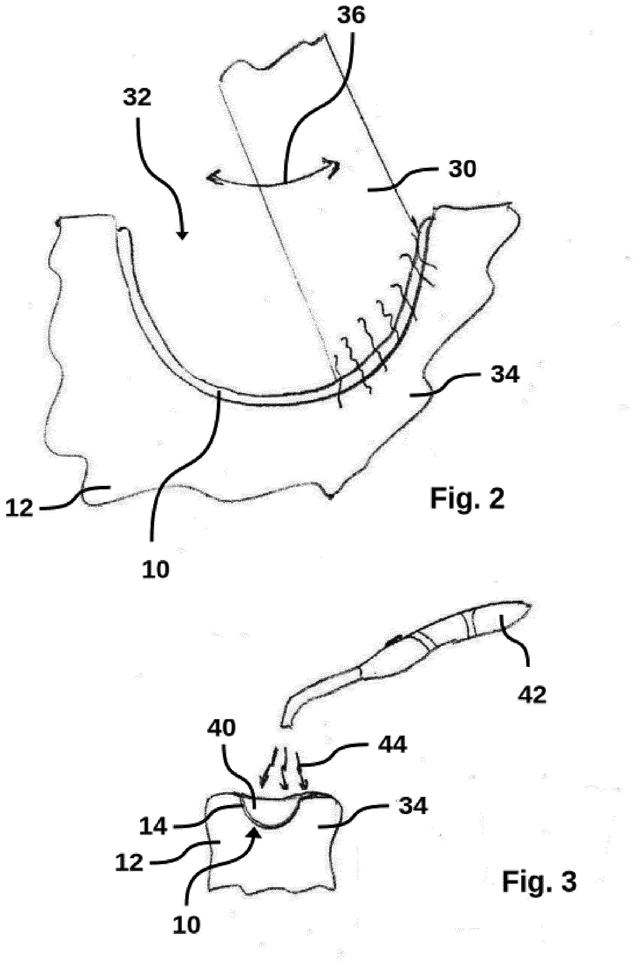

Aus Fig. 2 ist eine weitere Ausführungsform eines erfindungsgemäßen Systems in einer Detailvergrößerung ersichtlich. Die von dem Laser 16 abgegebene Strahlung 20 trifft in Form der Strahlung 30 auf die Oberfläche des Adhäsiv 14, das in der Kavität 32 aufgebracht ist. Die Strahlung 30 durchtritt die Adhäsivschicht 14 und dringt auch in das Zahnhartgewebe 34 ein.

Um eine möglichst gute Bestrahlung zu ermöglichen, wird das Handstück 24 so geschwenkt, dass die Strahlung 30 entsprechend dem Pfeil 36 möglichst senkrecht auf die Oberfläche der Adhäsivschicht 14 trifft. Die Oberfläche des Adhäsivs 14 liegt frei und ist für die Strahlung 30 daher frei zugänglich, da ein Dentalrestaurationsteil 40, s. Fig. 3, noch nicht aufgebracht ist.

Fig. 3 zeigt den Zustand, in welchem ein Dentalrestaurationsteil 40 bereits in die Kavität 32 eingebracht ist, das Dentalrestaurationsteil 40 also auf die Adhäsivschicht 14 aufgebracht worden ist.

Über ein Hand-Lichthärtgerät 42 wird das Adhäsiv 14 polymerisiert. Die abgegebene Polymerisationsstrahlung 44 durchtritt hierzu das für Laserstrahlung 44 im Bereich sichtbaren Lichts durchlässige Dentalrestaurationsteil 40.

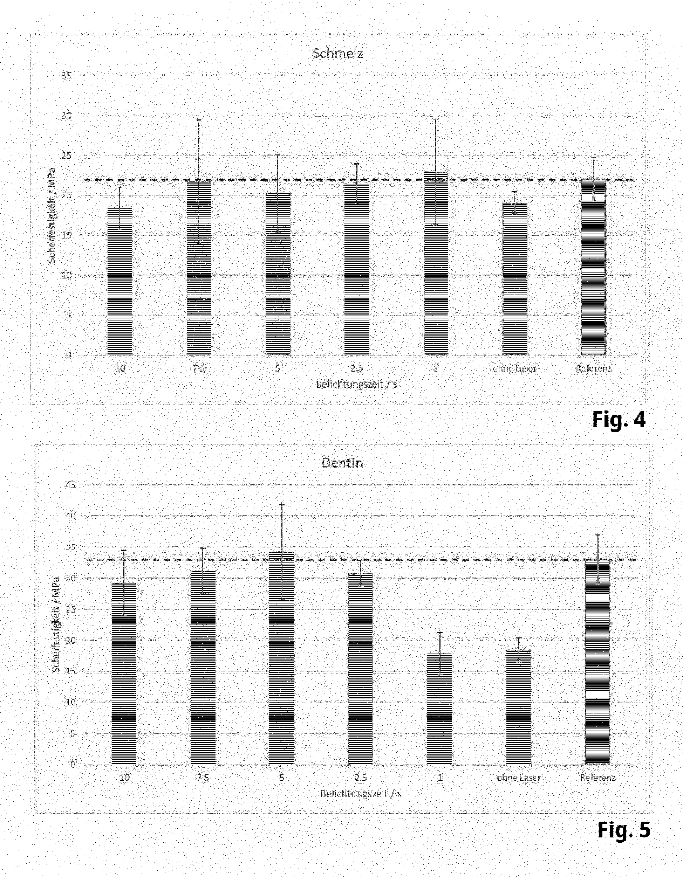

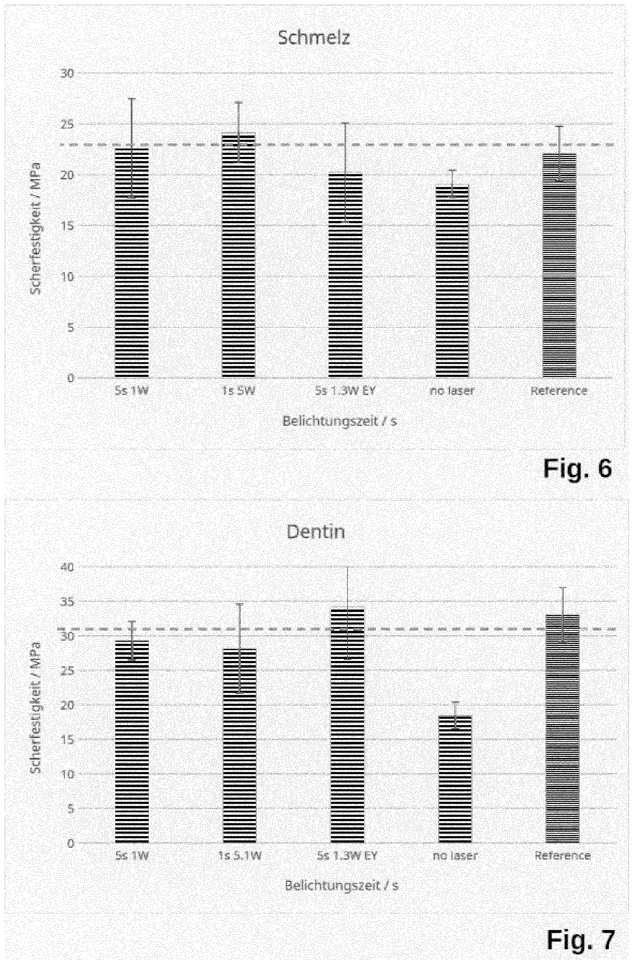

Fig. 4 zeigt ein Diagramm, geplottet basierend auf den Werten gemäß Tabelle 1 und Fig. 5 ein Diagramm, geplottet basierend auf den Werten aus Tabelle 2. Fig. 6 zeigt ein Diagramm, geplottet basierend auf den Werten gemäß Tabelle 3 und Fig. 7 ein Diagramm, geplottet basierend auf den Werten aus Tabelle 4.

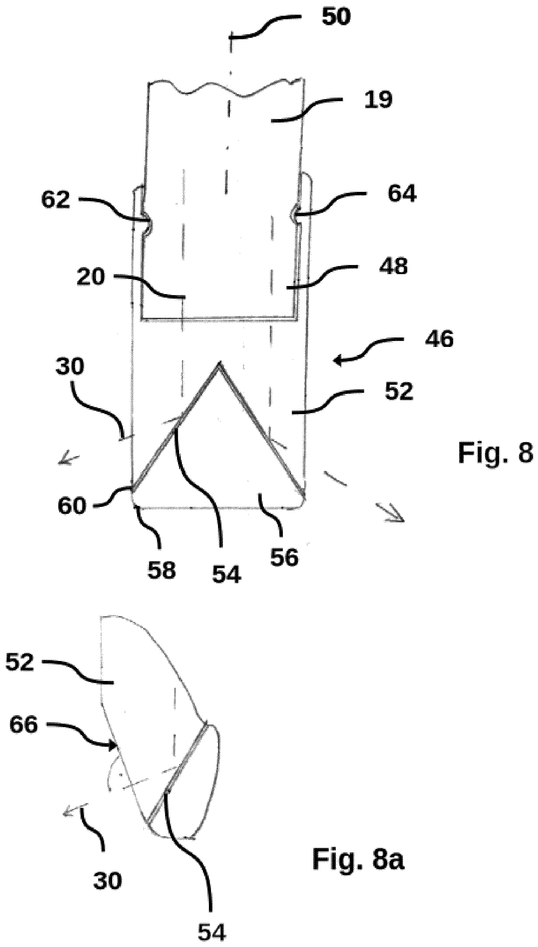

In einer modifizierten, in Fig. 8 dargestellten Ausführungsform ist der Behandlungs-Laser 16 mit einer Ablenkspitze 46 versehen. Die Ablenkspitze 46 ist in dieser Ausführungsform auf das distale Ende 48 des Lichtleiters 19 aufgesteckt. Alternativ kann die Ablenkspitze 46 auch einstückig mit dem Lichtleiter 19 ausgebildet sein, also in diesen integriert sein.

Die Ablenkspitze 46 ist koaxial zu einer Achse 50 des Lichtleiters 19 angeordnet. Sie besteht aus einem Körper 52 aus für die Strahlung 20 transparentem Material. An ihrem distalen Ende weist die Ablenkspitze 46 einen Innenkonus 54 auf. Dieser ist verspiegelt oder in anderer geeigneter Weise so ausgestaltet, dass er auftreffende Strahlung 20 reflektiert.

Die Strahlung 20 wird hierdurch von der Achse 50 abgelenkt und tritt seitlich aus der Ablenkspitze 46 als Strahlung 30 aus. Sie ist dafür bestimmt, auf das Adhäsiv 14, welches auf die Klebebasis 10 aufgebracht ist, zu treffen und dort als Behandlungs-Strahlung 30 zu wirken.

Diese Ausführungsform ist besonders für die Behandlung tiefer Kavitäten, die mit dem Adhäsiv 14 versehen sind, geeignet. Die Behandlungs-Strahlung 30 wird ringsum gestreut und abgelenkt, so dass auch schräge Flächen des Adhäsivs (vgl. Fig. 2) gleichmäßig mit Behandlungs-Strahlung 30 beaufschlagt werden.

Der Innenkegel 54 ist mit einem passenden Stopfen 56 aus weichem Material versehen, der bevorzugt eine hier als Endradius ausgebildete Abrundung 58 hat, Die Abrundung springt gegenüber einer scharfen umlaufenden Endkante 60 des Körpers 52 vor und deckt diese insofern ab. Der Stopfen 56 ist in den Innenkegel 54 eingeklebt. Mit der vorspringenden Abrundung 58 lassen sich Beschädigungen des Lasers 16 und Verletzungen des Patienten vermeiden.

Der Innenkegel 54 kann einen Kegelwinkel von 90 Grad haben. Dann tritt die Strahlung 30 senkrecht zur Achse 50 aus dem Körper 52 aus. In der dargestellten Ausführungsform beträgt der Kegelwinkel des Innenkegels 54 etwa 65 Grad. Die Strahlung 30 tritt dementsprechend leicht schräg nach vorne aus der Ablenkspitze 46 aus. Die Ablenkspitze 46 muss bei dieser Lösung daher nicht ganz so tief in eine tiefe Kavität eingeführt werden.

Es ist auch möglich, den Innenkegel 46 nur teilzuverspiegeln, also so, dass ein Teil der Strahlung, z.B. 50 %, reflektiert und der übrige Teil durchgelassen wird. Bei dieser Ausführungsform ist der Stopfen 56 für die Strahlung ebenfalls durchlässig.

Die abgegebene Strahlung trifft bei dieser Lösung dann nicht nur auf die Seitenwände, sondern auch auf den Grund der Kavität, was in vielen Fällen günstig ist.

Durch die Abnehmbarkeit der Ablenkspitze 46 ist es auch möglich, zwischen verschiedenen Emissionscharakteristiken zu wechseln.

Die Ablenkspitze 46 ist hier auf das distale Ende 48 des Lichtleiters 19 aufgesteckt. Hierzu weist dieses eine Ringnut 62 auf, die dafür bestimmt ist, dass ein Ringsteg 64 des Körpers 52 in diese eingreift und die Ablenkspitze 46 spielfrei auf dem Lichtleiter 19 lagert. Der Körper 52 besteht aus einem, ggf. etwas elastischen, strahlungsdurchlässigen Material, so dass beim Aufschieben des Körpers der Ablenkspitze 46 auf den Lichtleiter 19 der Ringsteg 64 in die Ringnut elastisch einrastet.

Alternativ kann an dieser Stelle eine Hülse aus einem elastischen Kunststoff vorgesehen sein, die sowohl den Körper 52 als auch das Ende 48 des Lichtleiters 19 übergreift.

Die Ablenkspitze 46 ist bei diesen beiden Ausführungsformen auf dem Lichtleiter 19 unverlierbar gehalten, aber mit Handkraft oder ggf. unter Zuhilfenahme eines geeigneten Werkzeugs wie einer Minizange abziehbar.

Bei einem 90 Grad unterschreitenden Kegelwinkel kann der Körper 52 der Ablenkspitze 46 auch gemäß Fig. 8a an seinem distalen Ende mit einem Außenkonus 66 versehen sein.

Fig. 8a zeigt einen Detailausschnitt zu Fig. 8. Der Außenkonus 66 ist hinsichtlich seines Kegelwinkels an den Kegelwinkel des Innenkonus 54 angepasst. Durch den Außenkonus 66 verlässt die Strahlung 30 den Körper 52 in zum Außenkonus 66 senkrechter Richtung, so dass Reflexionsverluste vermieden werden.

## Patentansprüche

1. System aus einem Laser und einem Dental-Adhäsiv, welcher Laser Laserstrahlung mit einer Wellenlänge von 1300 nm oder mehr, insbesondere zwischen 2750 nm und 3200 nm abgibt, und welches Adhäsiv zur Aufbringung auf eine vorbehandelte, insbesondere gereinigte, Klebebasis, die durch Zahnhartgewebe oder durch Prothesenmaterial gebildet ist, geeignet ist, wobei das Adhäsiv, insbesondere ohne Verblasen oder Agitieren, durch Applizieren von Laserstrahlung behandelbar ist, insbesondere aktivierbar ist, insbesondere, während das Adhäsiv frei von einem Dentalrestaurationsteil ist, und wobei auf dem behandelten, insbesondere aktivierten, Adhäsiv das Dentalrestaurationsteil befestigbar ist.

2. System nach Anspruch 1 oder, **dadurch gekennzeichnet, dass** der Laser (16) als Festkörperlaser mit mindestens einem Emissionsmaximum bei einer Wellenlänge zwischen 2750 nm und 3200 nm ausgebildet ist, und insbesondere als gepulster Laser, besonders bevorzugt als Er:YAG-Laser, ausgebildet ist.

3. System nach einem der vorhergehenden Ansprüche, **dadurch gekennzeichnet, dass** während des Laserbehandlungsschritts die Laserbehandlung für 0,5 sec oder mehr und 15 sec oder weniger, insbesondere 2 sec oder mehr und 8 sec oder weniger erfolgt, und zwar insbesondere gepulste Laser-Strahlung (20) mit einem Impuls/Pausen-Verhältnis zwischen 1:1 und 1:1000, insbesondere zwischen 1:5 und 1:200 und bevorzugt etwa 1:25 abgibt, oder, dass der Laser (16) als kontinuierlich emittierender Laser ausgebildet ist.

4. System nach einem der Ansprüche 1, **dadurch gekennzeichnet, dass** der Laser (16) als ungepulster Laser, insbesondere als Diodenlaser, ausgebildet ist.

5. System nach einem der Ansprüche 1, oder 4, **dadurch gekennzeichnet, dass** der Laser (16) mit mindestens einem Emissionsmaximum bei einer Wellenlänge zwischen etwa 1400 und 1600 nm ausgebildet ist

6. System nach einem der vorhergehenden Ansprüche, **dadurch gekennzeichnet, dass** das Adhäsiv (14) in einer Schichtdicke von 0,08 oder weniger mm, insbesondere von 0,03 mm oder weniger und insbesondere bevorzugt zwischen 0,01 bis 0,02 mm, aufgetragen ist.

7. System nach einem der vorhergehenden Ansprüche, **dadurch gekennzeichnet, dass** der Laser (16) ein Bakterienentfernungslaser ist, insbesondere ein solcher gemäß der EP 3 628 266 A1.

8. System nach einem der vorhergehenden Ansprüche, **dadurch gekennzeichnet, dass** der Laser (16) Strahlung (20) in einen Lichtleiter (19) abgibt, dessen distales Ende insbesondere abgekröpft und/oder biegsam ist, und dass die Strahlung (20) des Lasers (16) durch den Lichtleiter (19) dem Adhäsiv (14) und der Klebebasis (10) zuleitbar ist.

9. System nach einem der vorhergehenden Ansprüche, **dadurch gekennzeichnet, dass** ein Sensor (26) vorgesehen ist, der auf Strahlung im Wellenlängenbereich zwischen 1000 nm und 15000 nm anspricht und insbesondere ein Empfindlichkeitsmaximum oberhalb oder unterhalb des Emissionsmaximums des Lasers hat.

10. System nach Anspruch 9, **dadurch gekennzeichnet, dass** der Sensor (26) als Reflexionserfassungssensor ausgebildet ist, der insbesondere an einem Handstück (24) des Lasers (16) oder in dessen Nähe angebracht und gemeinsam mit diesem bewegbar ist.

11. System nach Anspruch 10, **dadurch gekennzeichnet, dass** der Sensor (26) beim Ausrichten der Strahlung des Lasers (16) auf die Klebebasis (10) und das Adhäsiv (14) von dieser abgegebenen Strahlung erfasst.

12. System nach einem der Ansprüche 9 bis 11, **dadurch gekennzeichnet, dass** eine Ziel-Lichtquelle (27) neben dem Laser (16) und im wesentlichen parallel zu diesen angebracht ist

13. System nach Anspruch 12, **dadurch gekennzeichnet, dass** die Ziel-Lichtquelle (27) Licht abgibt, das auf einem Fokusfleck (29) sichtbar ist.

14. System nach einem der vorhergehenden Ansprüche, **dadurch gekennzeichnet, dass** der Laser (16) eine Ablenkspitze (46) aufweist, mit welcher austretende Strahlung (20) gegenüber einer Achse (50) des Lasers (16) abgelenkt wird.
